# EUROPÄISCHE PATENTSCHRIFT

(11) **EP 1 779 900 B1**
(45) Veröffentlichungstag und Bekanntmachung des Hinweises auf die Patenterteilung: **24.12.2008**
(21) Anmeldenummer: 06022394.8
(22) Anmeldetag: 26.10.2006
(51) Int. Cl.: A61Q 17/04, A61K 8/63, A61K 8/67, A61K 31/56, A61Q 19/00

(54) **Kosmetische oder dermatologische Zubereitung zum Schutz vor Lichtalterung**
Cosmetic or dermatological composition for prevention of light-induced aging
Composition cosmétique ou dermatologique protégeant du vieillissement photo-induit

(30) Priorität: 29.10.2005 DE 102005051889
(43) Veröffentlichungstag der Anmeldung: 02.05.2007
(73) Patentinhaber: Apotheker Walter Bouhon GmbH, 90427 Nürnberg (DE)
(72) Erfinder: Neufang, Hartmut, 91056 Erlangen (DE); Till, Martina, 90765 Fürth (DE)
(74) Vertreter: Tergau & Pohl Patentanwälte

(56) Entgegenhaltungen:
- EP-A2- 0 472 225
- EP-A2- 1 000 613
- WO-A-01/70046
- DE-U1- 20 204 160
- US-A- 5 571 503
- US-A1- 2002 022 040
- US-A1- 2002 192 245
- DATABASE CAPLUS [Online] CHEMICAL ABSTRACTS SERVICE, COLUMBUS, OHIO, US; XP002415402 gefunden im STN Database accession no. 2002:04993 & JP 2002 080338 A (SHISEIDO CO.) 19. März 2002 (2002-03-19)

## Beschreibung

Die Erfindung betrifft die Verwendung einer Zusammensetzung zur Herstellung einer kosmetischen oder dermatologischen Zubereitung zum Schutz vor Lichtalterung. Die Erfindung betrifft weiter eine entsprechende kosmetische oder dermatologische Zubereitung als solche.

Aufgrund ihrer exponierten Lage und aufgrund ihrer Bedeutung als Schutz- und Abgrenzungsorgan gegenüber der Außenwelt ist die menschliche Haut im Gegensatz zu inneren Organen zusätzlich vielen exogenen Einflüssen ausgesetzt. Dabei gibt es neben kurzfristig wirkenden Einflüssen, wie z.B. bei einer Verletzung, insbesondere wiederholt auftretende und lang anhaltende Einflüsse, wie z.B. bei einer Lichtbelastung, insbesondere im UV-Bereich. Die Haut zeigt daher neben einer intrinsischen, d.h. chronologischen oder genetischen Alterung wie die übrigen Organe zusätzlich eine extrinsische, d.h. exogene und vorzeitige Alterung, die zum großen Teil lichtbedingt ist.

Die intrinsische Hautalterung verläuft für die gesamte Haut gleichermaßen. Eine chronologisch gealterte Haut ist bei feiner Faltenbildung dünn und atrophisch, und weist eine gleichmäßige Pigmentierung auf.

Bei der extrinsischen Hautalterung, die auch als Lichtalterung, aktinische Alterung oder vorzeitige Alterung bezeichnet wird, kommt es zu charakteristischen Veränderungen. Während die intrinsische Hautalterung zu einer Hautverdünnung oder Atrophie führt, kommt es bei der Lichtalterung in den meisten Fällen zu einer Dickenzunahme durch Zellproliferation. Weiter tritt vermehrte Faltenbildung durch Fehlvernetzungen im kollagenen Stützgewebe und eine vermehrte Melaninbildung mit fleckiger Pigmentierung auf.

Für die extrinsische oder lichtinduzierte Hautalterung sind eine Reihe von kosmetischen oder dermatologischen Zubereitungen bekannt, die eine derartige Hautalterung therapieren.

Beispielsweise ist aus der EP 0 859 592 B1 die Verwendung einer Kombination von Sterolen, Ubichinonen und Plastochinonen zur Behandlung lichtgealterter Haut bekannt. Auch wird es in der EP 0 859 592 B1 als bekannt beschrieben, dass Vitamin-A auf lichtinduzierte Strukturschäden der Haut wirkt.

Aus der EP 1 000 613 A2 ist weiter die Verwendung einer Vitamin-A und Phytosterole enthaltenden Zusammensetzung zur Behandlung einer Hautalterung bekannt, wobei durch die Verwendung der Zusammensetzung in der Haut der Anteil an sogenannten TIMP-Inhibitoren erhöht wird, die die kollagenabbauende Wirkung von Metalloproteinasen behindern. Die kollagenabbauenden Metalloproteinasen, so wird ausgeführt, sind als Folge des Alterungsprozesses in erhöhter Konzentration vorhanden.

Auch aus der EP 1 153 601 A1 ist eine Zusammensetzung zur Behandlung von Alterserscheinungen der Haut bekannt. Dort wird Vitamin-A, eingebunden in ein Fettsäuremonoglyzerid, als wirkungsvoll zur Verbesserung aufgetretener Hautalterungen wie Rauhigkeit oder Faltenbildung beschrieben.

Ferner ist aus der EP 0 229 561 B1 eine Zusammensetzung zur Bekämpfung der strahlungsbedingten und der chronologischen Hautalterung bekannt, wobei für die Zusammensetzung Vitamin-A, Phytosterole sowie Vitamin-E angegeben sind. Die als Sonnenschutzmittel angegebenen Zusammensetzungen beinhalten UV-A/B-Blocker, so dass Licht dieser Wellenlänge keine Hautschäden verursachen kann.

Weitere allgemeine Zusammensetzungen finden sich in der US 5,571,503, der DE 198 57 492 A1 und der DE 100 16 155 A1.

Aufgabe der Erfindung ist es, die Verwendung einer Zusammensetzung zur Herstellung einer kosmetischen oder dermatologischen Zubereitung anzugeben, die möglichst wirkungsvoll die Haut vor Lichtalterung schützt. Weiter ist es eine Aufgabe der Erfindung, eine entsprechende dermatologische oder kosmetische Zubereitung anzugeben.

Die erstgenannte Aufgabe wird erfindungsgemäß durch die Verwendung einer Zusammensetzung, die 0,02 bis 0,09 Gew.-% Vitamin-A, seine biochemischen Vorstufen oder seine Derivate, 0,01 bis 3 Gew.-% eines Phytosterols, seine biochemischen Vorstufen oder seine Derivate, 0,5 bis 3 Gew.-% Vitamin-E, seine biochemischen Vorstufen oder seine Derivate, sowie 1 bis 4 Gew.-% Harnstoff oder nach INCI-Norm UREA und 5 bis 8 Gew.-% Capryl/Caprinsäure-Triglycerid oder nach INCI-Norm Caprylic/Capric-Triglyceride umfasst, zur Herstellung einer kosmetischen oder dermatologischen Zubereitung zur Hemmung einer lichtinduzierten Genexpression für die Synthese von Kollagenasen in der Haut gelöst.

Durch umfangreiche Untersuchungen konnte die wirkungsvolle Hemmung der lichtinduzierten Genexpression für die Synthese von Kollagenasen einer Zusammensetzung mit Vitamin-A, einem Phytosterol und Vitamin-E in den angegebenen Mengenbereichen gezeigt werden. Diese Wirkung tritt ohne den Zusatz von herkömmlichen organischen und/oder anorganischen Lichtschutzfiltersubstanzen auf, so dass sich die angegebene Zusammensetzung zur Prävention oder Hemmung eines lichtinduzierten Kollagenabbaus in der Haut einsetzen lässt, ohne dass auf einen gewünschten Bräunungseffekt verzichtet werden muss. Durch den Einsatz der angegebenen Zusammensetzung wird bereits präventiv verhindert, dass durch Lichtwirkung Kollagenasen synthetisiert werden. Während bekannte Zusammensetzungen zur Behandlung von lichtinduzierten Hautalterungen oder zur Blockierung vorhandener Kollagenasen eingesetzt werden, greift die angegebene Zusammensetzung bereits in den Synthesemechanismus der Kollagenasen aktiv ein.

Die Erfindung beruht auf der Erkenntnis, dass Licht und insbesondere Licht im ultravioletten Bereich in Hautzellen zu einer Genexpression für die Synthese von Kollagenasen, d.h. Kollagen abbauenden Enzymen, führt. Die synthetisierten Kollagenasen, insbesondere sind dies Matrixmetalloproteinasen - kurz MMP -, führen zu einem Abbau des für die Hautelastizität und Hautstruktur verantwortlichen Kollagens. Hierdurch treten unreife kollagene Fasern auf, die die Fasern reifen Kollagens ersetzen. Dies ist ein wichtiges Charakteristikum der lichtgeschädigten Haut. Mit anderen Worten lösen Kollagenasen durch ihre Kollagen abbauende Wirkung einen Großteil der histologisch zu beobachtenden lichtinduzierten Hautveränderungen aus. Hierzu gehören z.B. die solare Elastose, die auch als so genannte Landmannshaut bezeichnet wird.

Umfangreiche Untersuchungen haben nun gezeigt, dass Vitamin-A, seine biochemischen Vorstufen oder seine Derivate, ein Phytosterol, seine biochemischen Vorstufen oder seine Derivate und Vitamin-E, seine biochemischen Vorstufen oder Derivate, aktiv in die Synthese der Kollagenasen eingreifen. Es hat sich überraschend gezeigt, dass durch die Verwendung der beschriebenen Substanzen die lichtinduzierte Synthese der Kollagenasen wirkungsvoll gehemmt werden kann. Die neuartige Wirkung beruht also darauf, dass lichtinduzierte Hautschäden gar nicht erst auftreten, sondern deren Entstehung durch Hemmung der Ursache wirkungsvoll behindert wird. Die unerwünschte Genexpression zur Synthese der Kollagenasen oder Matrixmetalloproteinasen wird behindert. Verschiedene durchgeführte in-vivo Versuchsreihen zeigen eine Hemmung der Expression von Matrixmetalloproteinasen um bis zu 65% für Phytosterole und um bis zu 82% für Vitamin-A. Insbesondere die Mischung von Vitamin-A, Phytosterolen und Vitamin-E zeigt eine hervorragende Wirkung zur Behinderung der MMP-Expression, wie es durch in-vivo Versuchsreihen gezeigt werden konnte.

Weiter ist der Zusammensetzung Harnstoff und Capryl/Caprinsäure-Triglycerid in der jeweils angegebenen Konzentration beigemengt. Durch die Kombination von Harnstoff und Capryl/Caprinsäure-Triglycerid wird insgesamt eine hautschonende, feuchtigkeitsregulierende und geschmeidig machende Wirkung erzielt.

Zur neuartigen Verwendung einer Zusammensetzung können insbesondere Retinoide der allgemeinen Formel 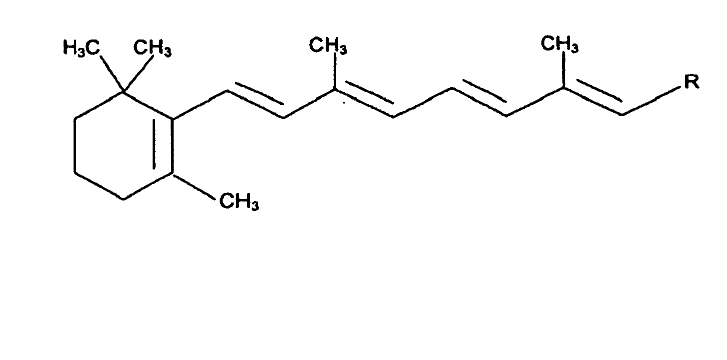 eingesetzt werden, wobei der Rest R ein Methanol, ein Formaldehyd, ein Methylacetat oder ein Methylpalmitat ist.

Hinsichtlich des Phytosterols kann ein Sterolderivat pflanzlicher Herkunft der Allgemeinen Formel 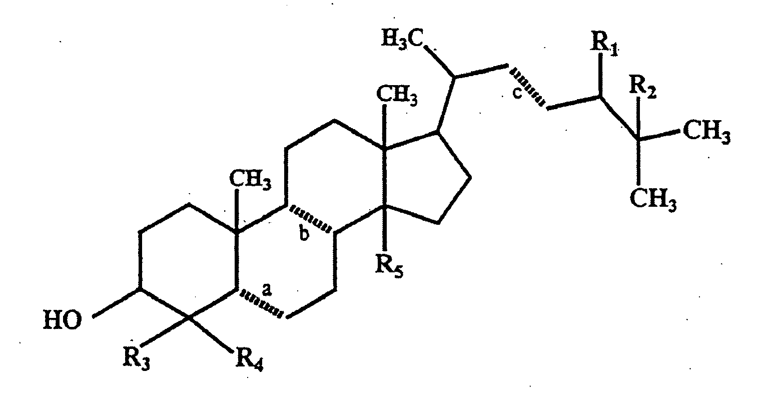 eingesetzt werden, wobei eine der beiden Bindungen a und b des Sterolderivats eine Einfach- und die andere eine Doppelbindung ist, wobei die Bindung c eine Einfach- oder eine Doppelbindung ist, wobei R₁ ein Wasserstoffatom, eine Alkylgruppe oder zusammen mit R₂ eine Doppelbindung ist, wobei R₂ ein Wasserstoffatom oder zusammen mit R₁ eine Doppelbindung ist, und wobei R₃, R₄ und R₅ jeweils ein Wasserstoffatom oder eine Methylgruppe sind.

Vorteilhafterweise wird für Vitamin-A in der Zusammensetzung ein Vitamin-A-Acetat eingesetzt, da dieses chemisch stabil ist. Gemäß INCI-Norm wird ein Vitamin-A-Acetat auch Retinyl-Acetate genannt.

Die INCI-Norm (International Nomenclature Cosmetic Ingredients: Internationale Nomenklatur für kosmetische Inhaltsstoffe) bezeichnet eine internationale Angaberichtlinie von Inhaltsstoffen von Kosmetika. Die Angabe der kosmetischen Inhaltsstoffe nach dem INCI-System ist in der EU seit 1997 gesetzlich vorgeschrieben und durch entsprechende Ländergesetze umgesetzt. Das INCI-System erlaubt eine eindeutige Identifikation kosmetischer Inhaltsstoffe. Da die Bezeichnungen gemäß INCI-System jedoch nicht immer den Namen der chemischen Verbindungen entsprechen, werden im Folgenden zur Bezeichnung kosmetischer Inhaltsstoffe soweit möglich sowohl die Namen der chemischen Verbindungen als auch die Namen gemäß INCI-Norm aufgeführt.

Für die Wirksamkeit des Vitamin-A beträgt dessen Anteil in der Zusammensetzung 0,02 bis 0,09 Gew.-%. Dies entspricht 500 bis 2500 internationalen Einheiten (IE) oder 172 bis 860 µg pro g Zubereitung. Bei guter Hautverträglichkeit ist hierbei eine wirksame Hemmung der Genexpression bezüglich der Kollagenasen gewährleistet.

In einer vorteilhaften Ausgestaltung wird als ein Phytosterol, d.h. als ein pflanzliches Sterol, ein Sitosterol, ein Campesterol, ein Stigmasterol, ein Brassicasterol oder eine Mischung hiervon eingesetzt. Die Phytosterole kommen in den Pflanzen frei, in Esther- oder in Glykosidform im Unverseifbaren der Fette vor.

Selbstverständlich ist es auch möglich, mehrere verschiedene Sterole, und insbesondere ein Cholesterol oder ein Lanosterin, in Kombination einzusetzen. Der Anteil der Phytosterole in der Zubereitung insgesamt beträgt zwischen 0,1 bis 3 Gew.-%.

Die lichtinduzierte Genexpression, die zur Synthese der Kollagenasen führt, wird insbesondere durch UVA-Bestrahlung induziert. Insofern ist eine vorteilhafte Verwendung der angegebenen Zusammensetzung zur Herstellung einer kosmetischen oder dermatologischen Zubereitung zur Hemmung einer durch UVA-Bestrahlung induzierten Genexpression zur Synthes von Kollagenasen.

Die kosmetische Zubereitung kann insbesondere eine O-W-Emulsion, eine W-O-Emulsion, eine Lotion oder eine Hautcreme ein.

Es hat sich gezeigt, dass durch die Zugabe von Vitamin-E die Wirkung hinsichtlich der Hemmung der beschriebenen Genexpression verbessert ist. Aufgrund der besseren chemischen Stabilität wird insbesondere ein Vitamin-E-Acetat oder nach INCI-Norm ein Tocopheryl Acetate eingesetzt. Dabei ist ein Anteil des Vitamin-E in der Zubereitung von 0,5 bis 3 Gew.-% wirksam.

Als zusätzlicher Wirkstoff kann der Zubereitung weiter Nikotinsäureamid oder nach INCI-Norm Niacinamide mit einem Anteil von 0,5 bis 2 Gew.-% beigefügt werden. Nikotinsäureamid ist auch als Vitamin-B3 bekannt.

Auch Ceramide können mit die Haut positiv beeinflussender Wirkung der Zubereitung mit einem Anteil von 0,05 bis 1 Gew.-% beigefügt werden.

Hinsichtlich des Feuchtehaushalts kann die Zubereitung weiter Natriumhyaluronat oder nach INCI-Norm Sodium Hyaloronate mit einem Anteil von 0,05 bis 0,25 Gew.-% enthalten.

Zusätzlich ist es günstig, wenn die Zubereitung weiter Glyzerin oder nach INCI-Norm Glyzerin mit einem Anteil zwischen 2 und 6 Gew.-% enthält.

Die zweitgenannte Aufgabe wird erfindungsgemäß durch eine kosmetische oder dermatologische Zubereitung gelöst, die 0,02 bis 0,09 Gew.-% Vitamin-A, seine biochemischen Vorstufen oder seine Derivate, 0,1 bis 3 Gew.-% eines Phytosterols, seine biochemischen Vorstufen oder seine Derivate, 0,5 bis 3 Gew.-% Vitamin-E, seine biochemischen Vorstufen oder seine Derivate sowie 1 bis 4 Gew.-% Harnstoff oder nach INCI-Norm UREA, 5 bis 8 Gew.-% Capryl/Caprinsäure-Triglyzerid oder nach INCI-Norm Caprylic/Capric Triglyceride, sowie Lipide, Konsistenzgeber, Emulgatoren, Polymere, Duftstoffe, Konservierungsstoffe und Rest Wasser enthält.

Neben der Hemmung der lichtinduzierten Genexpression bezüglich der Synthese von Kollagenasen weist eine derartige Zubereitung eine insgesamt hautschonende, feuchtigkeitsregulierende und geschmeidig machende Wirkung auf. Dies liegt insbesondere in der Kombination Harnstoff und Caprylic/Caprinsäure-Triglyzerid begründet.

Weitere vorteilhafte Ausgestaltungen finden sich in den Unteransprüchen 13 bis 22.

Für die geschmeidig machende Wirkung ist es vorteilhaft, als Lipide 1 bis 5 Gew.-% raffiniertes Jojobawachs oder nach INCl-Norm Simmondsia Chinensis sowie 2 bis 12 Gew.-% Ethyl-Hexylen-Stearat oder nach INCI-Norm Ethylhexyl-Stearate zu verwenden.

Hinsichtlich der Konsistenzgeber ist es vorteilhaft, wenn hierfür 1 bis 3 Gew.-% Glycerinmonostearat oder nach INCI-Norm Glycerylstearate, 0,5 bis 1,5 Gew.-% Glyceryl Stearate, 0,5 bis 1,5 Gew.-% Cetylstearylalkohol oder nach INCI-Norm Cetearyl-Alcohol und/oder 1,5 bis 2,5 Gew.-% Magnesiumstearat eingesetzt werden.

Als Polymere sind zweckmäßigerweise 0,1 bis 0,3 Gew.-% Carboxypolymethylen oder nach INCI-Norm Carbomer, 0,01 bis 0,3 Gew.-% Xanthan oder nach INCI-Norm Xanthan Gum und/oder 0,1 bis 0,2 Gew.-% AcrylsäureNinyl-Ester-Copolymer oder nach INCI-Norm Acrylates/Vinyl-Isodecanoate Crosspolymer eingesetzt.

Weiter von Vorteil ist es, wenn die Zubereitung 1 bis 3 Gew.-% eines Emulgators auf Zuckerbasis, insbesondere ein Polyglyceryl-3 Methylglukose Distearat nach INCI-Norm enthält.

Die Erfindung wird anhand der nachfolgenden Beispiele näher erläutert.

### Beispiel 1 (in vitro):

Es wird die Hemmung der UV-A-induzierten Genexpression am Beispiel der interstitiellen Kollagenase 1 (Matrixmetalloproteinase-1, MMP1) in humanen dermalen Fibroblasten untersucht. Die UV-A-induzierte Expression von MMP1 mRNS wird mittels Echtzeit PCR (Polymerase-Kettenreaktion) aus Zellen, die 24 Stunden nach der Bestrahlung mit 20 J/cm² geerntet worden sind, im Vergleich zu unbehandelten zeitgleichen Kontrollen bestimmt.

### a) Hemmung der UV-A-induzierten MMP1 mRNS-Expression in Abhängigkeit von der Vitamin-A-Konzentration:

Da Serum Vitamin-A enthält, werden die verwendeten Zellkulturen für 24 Stunden serum-depriviert. Zur Kontrolle wird die Genexpression an unbehandelten Zellen ohne und mit UV-A-Bestrahlung ermittelt. In einer Konzentrationsreihe von 0,1 bis 30 µM an zugesetztem Retinol wird jeweils die Genexpression der unbestrahlten und der mit UV-A bestrahlten Zellkulturen bestimmt.

Als Ergebnis stellt sich heraus, dass die mit Vitamin-A behandelten Zellkulturen eine Hemmung der MMP1-Expression zeigen, die bei einer Konzentration des Vitamin-A zwischen 10 und 25 µM bis zu 82% gegenüber der Kontrollmessung beträgt.

### b) Hemmung der UV-A-induzierten MMP1 mRNS Expression in Abhängigkeit von der Konzentration zugefügter Phytosterole:

Wiederum wird eine Kontrollmessung der Genexpression an unbehandelten Zellkulturen mit und ohne UV-A-Bestrahlung durchgeführt. Den Zellkulturen wird in einer Konzentrationsreihe zwischen 5 und 100µM Generol® R, ein käuflich erwerbbares Produkt der Cognis France SA zugefügt. Generol® R ist eine Mischung aus verschiedenen Phytosterolen und wird aus Raps gewonnen. Generol® R besteht zu 40 bis 60% aus Sitosterol, 25 bis 40% Campesterol, 7 bis 18% Brassicasterol und einem geringem Anteil an Stigmasterol. Zum Erreichen der beschriebenen Konzentrationsreihe wurde Generol® R entsprechend verdünnt. Für die behandelten Zellkulturen wurde jeweils die Genexpression mit und ohne UV-A-Bestrahlung bestimmt.

Als Ergebnis wurde eine über den gesamten untersuchten Konzentrationsbereich gleichmäßig verteilte Hemmung der MMP1 mRNS-Expression von bis zu 65% festgestellt.

### c) Hemmung der UV-A-induzierten MMP1 mRNS-Expression in Abhängigkeit von der Vitamin-E-Konzentration:

Wiederum wurde eine Kontrollmessung der unbehandelten Zellkulturen mit und ohne UV-A-Bestrahlung durchgeführt. In einer Konzentrationsreihe von 10 bis 75 µM an Vitamin-E-Sukzinat wurde die Genexpression jeweils mit und ohne UV-A-Bestrahlung bestimmt.

Als Ergebnis stellte sich heraus, dass in der untersuchten Konzentrationsreihe eine Hemmung der MMP1 mRNS-Expression um bis zu 75% gegenüber der Kontrollmessung erzielt wurde.

### Beispiel 2 (in vivo):

Es wurde die Hemmung der UV-A-induzierten Genexpression am Beispiel der interstitiellen Kollagenase 1 (Matrixmetalloproteinase-1, MMP1) in Biopsien aus UV-A-bestrahlten Probanden untersucht. Wiederum wurde die UV-A-induzierte Expression von MMP1 mRNS mittels Echtzeit PCR ermittelt. Die Messungen wurden hierzu an Biopsien durchgeführt, die 24 Stunden nach einer Bestrahlung mit 100J/cm² UV-A entnommen wurden, durchgeführt. Die Ergebnisse wurden mit unbehandelten zeitgleichen Kontrollen verglichen.

Insgesamt wurden vier Testpräparate eingesetzt.

Testpräparat 1 war eine dermatologische Zubereitung aus Neutralöl, Jojobawachs, TEGIN®, einem käuflich erwerbbaren Konsistenzgeber der Degussa, TEGO®-Care, einem käuflich erwerbbaren Emulgator der Degussa, Wasser, Synthalen® M, einem Carbomer der Degussa, Stabylen 30, einem käuflich erwerbbaren polymeren Emulgator der 3V-SIGMA, enthaltend Acrylsäure Vinyl-Ester-Copolymer oder nach INCI-Norm Acrylates-Vinyllisodecanoate Crosspolymer, Glyzerin, Harnstoff, Duftstoffe und Konservierungsmittel.

Als Testpräparat 2 wurden Testpräparat 1 Vitamin-E, Vitamin-A und Nicotinamid zugesetzt.

Als Testpräparat 3 wurde Testpräparat 1 GENEROL® sowie SK-influx®, ein käuflich erwerbbares Ceramide-Gemisch mit Xanthan der Degussa, zugesetzt.

Testpräparat 4 wurde aus Testpräparat 1 unter Hinzufügung der Zusätze gemäß Testpräparat 2 und Testpräparat 3 hergestellt, wobei zusätzlich Natriumhyaluronat beigemengt wurde.

An insgesamt zehn Probanden wurde die MMP1 mRNS-Expression mittels Echtzeit-PCR an den humanen Fibroblasten der Biopsien bestimmt. Als Vergleichsmessung dienten jeweils die unbehandelten zeitgleichen Kontrollen. Als Ergebnis ist folgendes festzuhalten:

Alle Probanden zeigen 24 Stunden nach der UV-A-Bestrahlung eine deutliche Aufregulation von MMP1 mRNS. Alle Testpräparate bewirken eine Hemmung der UV-A-induzierten MMP1 mRNS-Expression. Dabei steigt die Hemmung in Prozenten von Testpräparat 1 über Testpräparat 2 und Testpräparat 3 hin zu Testpräparat 4 im Mittel von 50% über 60% und 70% auf nahezu 80% an.

Das Hinzufügen von weiterem Vitamin-A in Testpräparat 2 führt zu einer deutlichen Zunahme der Hemmung der MMP1 mRNS-Expression. Eine ähnliche Wirkung haben die hinzugefügten Phytosterole des Testpräparats 3. Die größte Hemmung der MMP1 mRNS-Expression wurde mit Testpräparat 4 erzielt, welches eine Mischung aus Vitamin-A und Phytosterolen sowie Vitamin-E enthält. Der beste Schutz vor lichtinduziertem Kollagenabbau der Haut wird also mit Testpräparat 4 erzielt, welches bei einer Kombination von Vitamin-A und Phytosterolen auf einer Basis aus Jojobawachs, Harnstoff, Glyzerin sowie Emulgatoren, Stabilisatoren, Feuchthaltern und Konservierungsmitteln beruht.

Zur Herstellung der kosmetischen oder dermatologischen Zubereitung werden in einer ersten Phase A 7 Gew.-% Capryl/Caprinsäure-Triglycerid, 4 Gew.-% raffiniertes Jojobawachs, 6 Gew.-% Ethyl-Hexylen-Stearat, 2 Gew.-% Vitamin-E-Acetat, 2 Gew.-% Glyzereinmonostearat, 2 Gew.-% Generol® R, also der angegebenen Mischung aus Phytosterolen, 1,5 Gew.-% Polyglyceryl-3-Methylglukose-Distearat sowie 0,2 Gew.-% Vitamin-A-Acetat hergestellt. In erhitztes Wasser mit einem Gewichtsanteil von 59,75 Gew.-% werden 0,15 Gew.-% Carboxypolymethylen, 0,15 Gew.-% Acrylsäure Vinyl-Ester-Copolymer, 4 Gew.-% 86,5%igem Glyzerin, 2 Gew.-% Harnstoff sowie 0,1 Gew.-% Natriumhyaluronat eingebracht. Die entstehende Phase B wird weiter erhitzt und dieser unter leichtem Rühren die Phase A zugegeben.

Der entstandenen Mischphase aus Phasen A und B wird unter leichtem Rühren als Phase C 5 Gew.-% SK-influx-®, also einer Mischung aus Ceramiden, Carbomeren und Xanthan, zugegeben. Nach Homogenisieren der entstandenen Mischung wird diese auf ca. 40° abgekühlt.

Weiter wird eine Phase D aus 2 Gew.-% Wasser und 1 Gew.-% Nikotinamid hergestellt und homogenisiert. Die Phase D wird zugegeben. Abschließend werden Duftstoffe sowie Konservierungsmittel beigefügt.

## Patentansprüche

1. Verwendung einer Zusammensetzung, die 0,02 bis 0,09 Gew.-% Vitamin-A, seine biochemischen Vorstufen oder seine Derivate, 0,1 bis 3 Gew.-% eines Phytosterols, seine biochemischen Vorstufen oder seine Derivate, 0,5 Gew.-% bis 3 Gew.-% Vitamin-E, seine biochemischen Vorstufen oder seine Derivate, sowie 1 bis 4 Gew.-% Harnstoff oder nach INCI-Norm UREA und 5 bis 8 Gew.-% Capryl/Caprinsäure-Triglycerid oder nach INCI-Norm Caprylic/Capric-Triglyceride umfasst, zur Herstellung einer kosmetischen oder dermatologischen Zubereitung zur Hemmung einer lichtinduzierten Genexpression für die Synthese einer Kollagenase in der Haut.

2. Verwendung nach Anspruch 1,
wobei ein Vitamin-A-Acetat oder nach INCI-Norm ein Retinyl Acetate umfasst ist.

3. Verwendung nach Anspruch 1 oder 2,
wobei das Phytosterol ein Sitosterol, ein Campesterol, ein Stigmasterol, ein Brassicasterol oder eine Mischung hiervon ist.

4. Verwendung nach einem der vorhergehenden Ansprüche,
zur Herstellung einer kosmetischen oder dermatologischen Zubereitung zur Hemmung einer durch UV-A-Bestrahlung induzierten Genexpression für die Synthese einer Kollagenase in der Haut.

5. Verwendung nach einem der vorhergehenden Ansprüche,
wobei die kosmetische Zubereitung eine O/W-Emulsion, eine W/O-Emulsion, eine Lotion oder eine Hautcreme ist.

6. Verwendung nach einem der vorhergehenden Ansprüche,
wobei die Zubereitung ein Vitamin-E-Acetat oder nach INCI-Norm ein Tocopheryl Acetate enthält.

7. Verwendung nach einem der vorhergehenden Ansprüche,
wobei die Zubereitung weiter Nikotinsäureamid oder nach INCI-Norm ein Niacinamide mit einem Anteil von 0,5 bis 2 Gew.-% enthält.

8. Verwendung nach einem der vorhergehenden Ansprüche,
wobei die Zubereitung weiter gemäß INCI-Norm Ceramide mit einem Anteil von 0,05 bis 1 Gew.-% enthält.

9. Verwendung nach einem der vorhergehenden Ansprüche,
wobei die Zubereitung weiter Natriumhyaluronat oder nach INCI-Norm Sodium Hyaluronate mit einem Anteil von 0,05 bis 0,25 Gew.-% enthält.

10. Verwendung nach einem der vorhergehenden Ansprüche,
wobei die Zubereitung weiter Glyzerin oder nach INCI-Norm Glycerin mit einem Anteil von 2 bis 6 Gew.-% enthält.

11. Kosmetische oder dermatologische Zubereitung, die 0,02 bis 0,09 Gew.-% Vitamin-A, seine biochemischen Vorstufen oder seine Derivate, 0,1 bis 3 Gew.-% eines Phytosterols, seine biochemischen Vorstufen oder seine Derivate, 0,5 bis 3 Gew.-% Vitamin-E, seine biochemischen Vorstufen oder seine Derivate, sowie 1 bis 4 Gew.-% Harnstoff oder nach INCI-Norm UREA, 5 bis 8 Gew.-% Capryl/Caprinsäure-Triglycerid oder nach INCI-Norm Caprylic/Capric Triglyceride, sowie Lipide, Konsistenzgeber, Emulgatoren, Polymere, Duftstoffe, Konservierungsstoffe, und Rest Wasser enthält.

12. Zubereitung nach Anspruch 11,
die als Lipide 1 bis 5 Gew.-% raffiniertes Jojobawachs oder nach INCI-Norm Simmondsia Chinensis und 2 bis 12 Gew.-% Ethyl-Hexylen-Stearat oder nach INCI-Norm Ethylhexyl Stearate enthält.

13. Zubereitung nach Anspruch 11 oder 12,
wobei die Vitamin-A Vorstufe ein Vitamin-A-Acetat oder nach INCI-Norm Retinyl Acetate ist.

14. Zubereitung nach einem der Ansprüche 11 bis 13,
wobei das Phytosterol ein Sitosterol, ein Campesterol, ein Stigmasterol, und/oder ein Brassicasterol oder eine Mischung hiervon ist.

15. Zubereitung nach einem der Ansprüche 11 bis 14,
die Vitamin-E-Acetat oder nach INCI-Norm Tocopheryl Acetate enthält.

16. Zubereitung nach einem der Ansprüche 11 bis 15,
die als Wirkstoff weitere Vitamine, insbesondere 0,5 bis 2 Gew-% Nikotinsäureamid oder nach INCI-Norm Niacinamide und/oder 0,5 bis 3 Gew-% Panthenol, enthält.

17. Zubereitung nach einem der Ansprüche 11 bis 16,
die als Wirkstoffe 0,05 bis 1 Gew.-% Ceramide und/oder 0,05 bis 0,25 Gew-% Natriumhyaluronat oder nach INCI-Norm Sodium Hyaluronate enthält.

18. Zubereitung nach einem der Ansprüche 11 bis 17,
die weiter 2 bis 6 Gew-% Glyzerin oder nach INCI-Norm Glycerin enthält.

19. Zubereitung nach einem der Ansprüche 11 bis 18,
die als Konsistenzgeber 1 bis 3 Gew.-% Glyzerinmonostearat oder nach INCI-Norm Glyceryl Stearate, 0,5 bis 1,5 Gew.-% Cetylstearylalkohol oder nach INCI Norm Cetearyl Alcohol und/oder 1,5 bis 2,5 Gew.-% Magnesiumstearat enthält.

20. Zubereitung nach einem der Ansprüche 11 bis 19,
die als Polymere 0,1 bis 0,3 Gew.-% Carboxypolymethylen oder nach INCI Norm Carbomer, 0,01 bis 3 Gew.-% Xanthan oder nach INCI-Norm Xanthan Gum und/oder 0,1 bis 0,2 Gew.-% Acrylsäure/Vinyl-Ester-Copolymer oder nach INCI-Norm Acrylates/Vinyl Isodecanoate Crosspolymer enthält.

21. Zubereitung nach einem der Ansprüche 11 bis 20,
die 1 bis 3 Gew.-% eines Emulgators auf Zuckerbasis, insbesondere ein Polyglyceryl-3 Methylglukose Distearat nach INCI-Norm, enthält.

## Claims

1. Use of a composition which comprises 0.02 to 0.09% by weight of vitamin A, its biochemical precursors or its derivatives, 0.1 to 3% by weight of a phytosterol, its biochemical precursors or its derivatives, 0.5% by weight to 3% by weight of vitamin E, its biochemical precursors or its derivatives, and also 1 to 4% by weight of urea and 5 to 8% by weight of caprylic/capric triglyceride for the preparation of a cosmetic or dermatological preparation for inhibiting a photoinduced gene expression for the synthesis of a collagenase in the skin.

2. Use according to Claim 1,
where a vitamin A acetate or, according to INCI standard, a Retinyl Acetate, is comprised.

3. Use according to Claim 1 or 2,
where the phytosterol is a sitosterol, a campesterol, a stigmasterol, a brassicasterol or a mixture thereof.

4. Use according to one of the preceding claims,
for the preparation of a cosmetic or dermatological preparation for inhibiting a gene expression induced by UV-A irradiation for the synthesis of a collagenase in the skin.

5. Use according to one of the preceding claims,
where the cosmetic preparation is an O/W emulsion, a W/O emulsion, a lotion or a skin cream.

6. Use according to one of the preceding claims,
where the preparation comprises a vitamin E acetate or, according to INCI standard, a Tocopheryl Acetate.

7. Use according to one of the preceding claims,
where the preparation further comprises nicotinamide or, according to INCI standard, a Niacinamide in a fraction of from 0.5 to 2% by weight.

8. Use according to one of the preceding claims,
where the preparation further comprises, according to INCI standard, Ceramide in a fraction of from 0.05 to 1% by weight.

9. Use according to one of the preceding claims,
where the preparation further comprises sodium hyaluronate in a fraction of from 0.05 to 0.25% by weight.

10. Use according to one of the preceding claims,
where the preparation further comprises glycerol or, according to INCI standard, Glycerin in a fraction of from 2 to 6% by weight.

11. Cosmetic or dermatological preparation which comprises 0.02 to 0.09% by weight of vitamin A, its biochemical precursors or its derivatives, 0.1 to 3% by weight of a phytosterol, its biochemical precursors or its derivatives, 0.5 to 3% by weight of vitamin E, its biochemical precursors or its derivatives, and 1 to 4% by weight of urea, 5 to 8% by weight of caprylic/capric triglyceride, and also lipids, consistency regulators, emulsifiers, polymers, fragrances, preservatives, and remainder water.

12. Preparation according to Claim 11,
which comprises, as lipids, 1 to 5% by weight of refined jojoba wax or, according to INCI standard, Simmondsia Chinensis and 2 to 12% by weight of ethyl hexylene stearate or, according to INCI standard, Ethylhexyl Stearate.

13. Preparation according to Claim 11 or 12,
where the vitamin A precursor is a vitamin A acetate or, according to INCI standard, Retinyl Acetate.

14. Preparation according to one of Claims 11 to 13,
where the phytosterol is a sitosterol, a campesterol, a stigmasterol and/or a brassicasterol or a mixture thereof.

15. Preparation according to one of Claims 11 to 14,
which comprises vitamin E acetate or, according to INCI standard, Tocopheryl Acetate.

16. Preparation according to one of Claims 11 to 15,
which comprises, as active ingredient, further vitamins, in particular 0.5 to 2% by weight of nicotinamide or, according to INCI standard, Niacinamide and/or 0.5 to 3% by weight of panthenol.

17. Preparation according to one of Claims 11 to 16,
which comprises, as active ingredients, 0.05 to 1% by weight of ceramides and/or 0.05 to 0.25% by weight of sodium hyaluronate.

18. Preparation according to one of Claims 11 to 17,
which further comprises 2 to 6% by weight of glycerol or, according to INCI Standard, Glycerin.

19. Preparation according to one of Claims 11 to 18,
which comprises, as consistency regulator, 1 to 3% by weight of glycerol monostearate or, according to INCI standard, Glyceryl Stearate, 0.5 to 1.5% by weight of cetylstearyl alcohol or, according to INCI standard, Cetearyl Alcohol and/or 1.5 to 2.5% by weight of magnesium stearate.

20. Preparation according to one of Claims 11 to 19,
which comprises, as polymers, 0.1 to 0.3% by weight of carboxypolymethylene or, according to INCI standard, Carbomer, 0.01 to 3% by weight of xanthan or, according to INCI Standard, Xanthan Gum and/or 0.1 to 0.2% by weight of acrylic acid/vinyl ester copolymer or, according to INCI Standard, Acrylates/Vinyl Isodecanoate Crosspolymer.

21. Preparation according to one of Claims 11 to 20,
which comprises 1 to 3% by weight of an emulsifier based on sugar, in particular a Polyglyceryl-3 Methylglucose Distearate according to INCI standard.

## Revendications

1. Utilisation d'une composition qui comprend 0,02 à 0,09 % en poids de vitamine A, ses précurseurs biochimiques ou ses dérivés, 0,1 à 3 % en poids d'un phytostérol, ses précurseurs biochimiques ou ses dérivés, 0,5 % en poids à 3 % en poids de vitamine E, ses précurseurs biochimiques ou ses dérivés, ainsi que 1 à 4 % en poids d'urée ou Urea selon la norme INCI et 5 à 8 % en poids de triglycéride caprylique/ caprique ou Caprylic/Capric Triglyceride selon la norme INCI, pour la fabrication d'une préparation cosmétique ou dermatologique destinée à inhiber une expression génique photo-induite pour la synthèse d'une collagénase dans la peau.

2. Utilisation selon la revendication 1, où est également compris un acétate de vitamine A ou Retinyl Acetate selon la norme INCI.

3. Utilisation selon la revendication 1 ou 2, où le phytostérol est un sitostérol, un campestérol, un stigmastérol, un brassicastérol ou un mélange de ces derniers.

4. Utilisation selon une des revendications précédentes, pour la fabrication d'une préparation cosmétique ou dermatologique destinée à inhiber une expression génique induite par le rayonnement UV-A pour la synthèse d'une collagénase dans la peau.

5. Utilisation selon l'une des revendications précédentes, où la préparation cosmétique est une émulsion huile-dans-l'eau, une émulsion eau-dans-l'huile, une lotion ou une crème cutanée.

6. Utilisation selon l'une des revendications précédentes, où la préparation contient un acétate de vitamine E ou Tocopheryl Acetate selon la norme INCI.

7. Utilisation selon l'une des revendications précédentes, où la préparation contient en outre d'un amide nicotinique ou Niacinamide selon la norme INCI dans une proportion de 0,5 à 2 % en poids.

8. Utilisation selon l'une des revendications précédentes, où la préparation contient en outre des céramides selon la norme INCI dans une proportion de 0,05 à 1 % en poids.

9. Utilisation selon l'une des revendications précédentes, où la préparation contient en outre du hyaluronate de sodium ou sodium hyaluronate selon la norme INCI dans une proportion de 0,05 à 0,25 % en poids.

10. Utilisation selon l'une des revendications précédentes, où la préparation contient en outre du glycérol ou Glycerin selon la norme INCI dans une proportion de 2 à 6 % en poids.

11. Préparation cosmétique ou dermatologique qui comprend 0,02 à 0,09 % en poids de vitamine A, ses précurseurs biochimiques ou ses dérivés, 0,1 à 3 % en poids d'un phytostérol, ses précurseurs biochimiques ou ses dérivés, 0,5 à 3 % en poids de vitamine E, ses précurseurs biochimiques ou ses dérivés, ainsi que 1 à 4 % d'urée ou Urea selon la norme INCI, 5 à 8 % en poids de triglycéride d'acide caprylique/ caprique ou Caprylic/Capric Triglycéride selon la norme INCI, ainsi que des lipides, des épaississants, des émulsifiants, des polymères, des parfums, des agents de conservation et le reste étant de l'eau.

12. Préparation selon la revendication 11, qui contient comme lipide 1 à 5 % en poids de cire de jojoba raffinée ou Simmondsia Chinensis selon la norme INCI et 2 à 12 % en poids de stéarate d'éthylhexylène ou Ethylhexyl Stearate selon la norme INCI.

13. Préparation selon la revendication 11 ou 12, où le précurseur de vitamine A est un acétate de vitamine A ou Retinyl Acétate selon la norme INCI.

14. Préparation selon l'une des revendications 11 à 13, où le phytostérol est un sitostérol, un campestérol, un stigmastérol et/ou un brassicastérol ou un mélange de ces derniers.

15. Préparation selon l'une des revendications 11 à 14, qui contient de l'acétate de vitamine E ou Tocopheryl Acétate selon la norme INCI.

16. Préparation selon l'une des revendications 11 à 15, qui contient, comme principe actif, d'autres vitamines, notamment 0,5 à 2 % en poids d'amide nicotinique ou Niacinamide selon la norme INCI et/ou 0,5 à 3 % en poids de panthénol.

17. Préparation selon l'une des revendications 11 à 16, qui contient comme principes actifs 0,05 à 1 % en poids de céramide et/ou 0,05 à 0,25 % en poids de hyaluronate de sodium ou sodium hyaluronate selon la norme INCI.

18. Préparation selon l'une des revendications 11 à 17, qui contient en outre 2 à 6 % en poids de glycérol ou Glycerin selon la norme INCI.

19. Préparation selon l'une des revendications 11 à 18, qui contient comme épaississant 1 à 3 % en poids de monostéarate de glycérol ou Glyceryl Stearate selon la norme INCI, 0,5 à 1,5 % en poids d'alcool cétylstéarylique ou Cetearyl Alcohol selon la norme INCI et/ou 1,5 à 2,5 % en poids de stéarate de magnésium.

20. Préparation selon l'une des revendications 11 à 19, qui contient comme polymères 0,1 à 0,3 % en poids de carboxypolyméthylène ou Carbomer selon la norme INCI, 0,01 à 3 % en poids de xanthane ou Xanthan Gum selon la norme INCI et/ou 0,1 à 0,2 % en poids de copolymère acide acrylique/ester vinylique ou Acrylates/Vinyl Isodecanoate Crosspolymer selon la norme INCI.

21. Préparation selon l'une des revendications 11 à 20, qui contient 1 à 3 % en poids d'un émulsifiant à base de sucre, notamment un Polyglyceryl-3 Methylglucose Distearate selon la norme INCI.
